# EUROPÄISCHE PATENTSCHRIFT

(11) **EP 0 371 910 B1**
(45) Veröffentlichungstag und Bekanntmachung des Hinweises auf die Patenterteilung: **26.04.1995**
(21) Anmeldenummer: 89730216.2
(22) Anmeldetag: 01.12.1989
(51) Int. Cl.: A61B 17/58

(54) **Marknagel**
Intramedullary nail
Clou intramédullaire

(30) Priorität: 01.12.1988 DE 3840798
(43) Veröffentlichungstag der Anmeldung: 06.06.1990
(73) Patentinhaber: Johnson & Johnson Professional Products GmbH, 22848 Norderstedt (DE)
(72) Erfinder: Kranz, Curt, Dr., D-1000 Berlin 62 (DE); Seiler, Hanns, Prof. Dr. med., D-6633 Wadgassen (DE)
(74) Vertreter: UEXKÜLL & STOLBERG

(56) Entgegenhaltungen:
- EP-A- 0 260 222
- WO-A-88/01849
- DE-A- 3 537 318
- GB-A- 2 146 535

## Beschreibung

Die Erfindung betrifft einen Marknagel der im Oberbegriff des Anspruchs 1 angegebenen Art.

Die von Küntscher 1940 eingeführte Marknagelung stellt heute eine häufig angewandte Methode zur operativen Behandlung von Frakturen langer Röhrenknochen dar. Gegenüber anderen Verfahren sind vor allem folgende Vorzüge hervorzugeben: feste Fixierung der Bruchenden durch innere Schienung; keine operative Eröffnung der Frakturstelle, geringe Infektionsgefahr, kurze Liegedauer, frühzeitiges Aufstehen des Patienten ohne Gips sowie Nichtnotwendigkeit einer Nachbehandlung, da Muskelatrophien oder Bewegungseinschränkungen nicht auftreten.

Die Verriegelung des Marknagels ist bei allen Diaphysenfrakturen notwendig, die nach normaler Marknagelung instabil bleiben. Bei Pseudarthrosen kann je nach Lokalisation des Bruches, das proximale oder distale Fragment verriegelt werden.

Als problematisch bei der Verriegelungsbohrung erweist sich das Anvisieren der tiefer gelegenen Schraubenlöcher des Nagels, da dieser sich beim Eintreiben in das Knocheninnere verbiegen kann. Zum wiederauffinden der Schraubenlöcher muß deshalb ein Röntgenmanipulator eingesetzt werden.

Ein aus der DE-A-35 37 318 bekannter Marknagel ist auf der gesamten Rückseite mit einem für Marknägel üblichen Schlitz versehen und weist auf der Vorderseite zwei als längliche Schlitze ausgebildete Schraubenlöcher für die Verriegelungsschrauben auf, wobei die Breite dieser Schlitze der Breite der einzubringenden Verriegelungsschrauben angepaßt sind. Dadurch, daß die Schraubenlöcher als längliche Schlitze ausgebildet sind, wird das Einbringen der Verriegelungsschrauben ohne Röntgenmanipulator ermöglicht. Der Verriegelungsnagel kann an den Verriegelungsschrauben entlang der Schlitze gleiten. Um den Marknagel auch in Längsrichtungzu verriegeln, müssen wenigstens zwei Schrauben endständig in den Schlitzen gesetzt werden, von denen wenigstens eine positionsgenau eingebracht werden muß.

Es ist Aufgabe der vorliegenden Erfindung, einen verriegelbaren Marknagel zu schaffen, der die Verriegelung des distalen, tiefer gelegenen Bereichs des Marknagels ohne den Einsatz eines Röntgenmanipulators oder Zielgerätes noch weiter vereinfacht.

Erfindungsgemäß wird diese Aufgabe dadurch gelöst, daß der Marknagel nur im Bereich seiner Nagelspitze aus einem resorbierbaren Material besteht. Dadurch ist es möglich, auf eine Vorbohrung des Marknagels in jenem tiefer gelegenen Bereich, der aus dem resorbierbaren Material besteht, zu verzichten. Das Bohren der Löcher für die Verriegelungsschrauben erfolgt nach dem Eintreiben des Marknagels. Die dabei anfallenden Späne sind nicht schädlich, da sie in körpereigene Substanz umgesetzt weden. Da keine Lokalisation der Schraubenlöcher im Marknagel mehr erforderlich ist, entfällt der Einsatz eines Röntgenmanipulators oder Zielgeräts.

Bei bestimmten Frakturen des Femurs und der Tibia kann auch eine Verriegelung an dem oberen, nicht aus resorbierbarem Material bestehenden Teil des Marknagels, d.h. am Nagelkopf, erforderlich sein. Diese Verschraubung ist jedoch weitgehend unproblematisch, da sich die Position der entsprechenden dann vorgefertigten Bohrlöcher aus der Ausrichtung des Marknagels eindeutig ergibt.

Vorteilhaft erweist sich ferner die Ausführung mindestens einer der Verriegelungsschrauben aus resorbierbarem Material. Insbesondere die zur Verriegelung der resorbierbaren Nagelspitze eingesetzten Schrauben sollten selbst resorbierbar sein, weil dadurch ein postoperativer Eingriff zum Entfernen dieser Verriegelungsschrauben entfällt. Als resorbierbares Material bietet sich beispielsweise das bewährte Polylactid an.

Die sichere Zusammenfügung der beiden Teile des Marknagels erfolgt entsprechend einer vorteilhaften Ausführung der Erfindung mittels einer nichtrostenden Hülse, die über die zu verbindenden Enden geschoben und festgeklemmt wird.

Zweckmäßigerweise richtet sich die Länge des resorbierbaren Teils des Marknagels nach der vorgesehenen Lage der Verriegelungen. Damit wird in jedem Fall hohe Stabilität und Haltbarkeit bis zum vollständigen Verheilen des Bruches gewährsleistet.

Andererseits ist die Gesamtlänge des Marknagels nicht als besonders kritisch anzusehen, denn während ein metallischer Teil des Marknagels so kurz wie möglich gehalten sein sollte, kann der resorbierbare Teil des Nagels auch länger als unbedingt nötig sein, ohne Komplikationen zu verursachen. Nach Verheilung des Bruches werden nur die metallischen Teile des Implantats entfernt. Der resorbierbare Teil ist inzwischen durch die allmähliche Umwandlung in körpereigene Substanz soweit geschrumpft, daß das mechanische Verbindungselement, beispielsweise in Form einer Hülse, unwirksam geworden ist und somit nur noch der metallische Teil des Marknagels entfernt zu werden braucht.

Da als Hilfsmittel zum Eintreiben des Marknagels ein Führungsspieß verwendet wird, ist der Nagel in seiner ganzen Länge zentrisch durchbohrt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand einer Zeichnung näher dargestellt.

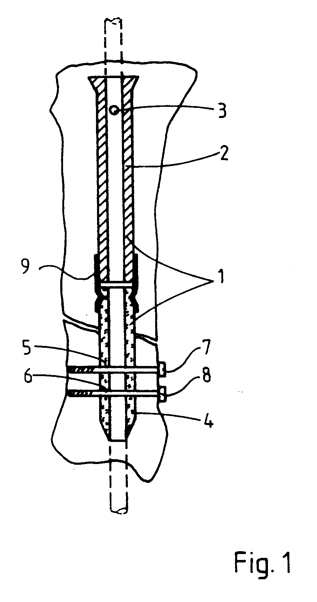

Die Figur 1 zeigt schematisch eine Schnittdarstellung des fixierbaren Marknagels mit andeutungsweise dargestellter distaler Fraktur.

Ein zentrisch durchbohrter Marknagel 1 wird unter Zuhilfenahme eines Markraumbohrers und eines Führungsspießes (gestrichelt dargestellt) in das Knochenmark eingetrieben, wobei der Führungsspieß anschließend wieder herausgezogen wird. Der Marknagel 1 ist an seiner Kopfseite 2 mit einem Loch 3 versehen, in welchem nach Anvisieren mit dem Zielgerät eine Verriegelungsschraube eingesetzt wird. Die aus resorbierbarem Material bestehende Nagelspitze 4 wird erst nach dem Eintreiben des Marknagels 1 an den Stellen 5 und 6 angebohrt. Die beim Bohren anfallenden Späne sind aufgrund der Materialeigenschaften des resorbierbaren Materials völlig unproblematisch und brauchen nicht entfernt zu werden.

Das Einschrauben der ebenfalls aus resorbierbarem Material bestehenden Verriegelungsschrauben 7 und 8 kann nunmehr ohne weiteres und ohne besonderes Ausrichten der genauen Schraubposition vorgenommen werden, da keine vorgebohrten Marknagellöcher mit Durchstrahlungsmitteln aufgesucht werden müssen.

Der Nagelkopf 2 und die Nagelspitze 4 sind durch eine dünnwandige Hülse 9 miteinander formfest verbunden. Diese Hülse 9 wird bei Herstellung des Marknagels über die beiden zu verbindenden Marknagelteile geschoben und verpreßt. Vorzugsweise bestehen der resorbierbare Teil des Marknagels sowie die Verriegelungsschrauben 7 und 8 aus Polylactid und die Hülse aus Titan oder einer Titanlegierung.

Die Verbindungsstelle der beiden Teile des Marknagels befindet sich nur wenig oberhalb des für die Verriegelungsschrauben 7 und 8 vorgesehenen Bereiches. Stabilität bis zum Verwachsen der Knochenfragmente ist damit gewährleistet. Nach der endgültigen Heilung werden alle Teile des Implantats entfernt, außer der resorbierbaren Marknagelspitze sowie den resorbierbaren Verriegelungsschrauben 7 und 8, welche sich inzwischen bereits weitgehend aufgelöst haben.

## Patentansprüche

1. Marknagel (1), der mittels Verriegelungsschrauben (7, 8) fixierbar ist, zur operativen Behandlung von Frakturen langer Röhrenknochen,
**dadurch gekennzeichnet,**
daß der Marknagel (1) nur im Bereich seiner Nagelspitze (4) aus einem resorbierbaren Material besteht.

2. Marknagel (1) nach Anspruch 1, **dadurch gekennzeichnet,** daß mindestens eine der Verriegelungsschrauben (7, 8) aus resorbierbarem Material besteht.

3. Marknagel (1) nach einem der vorhergehenden Ansprüche, **dadurch gekennzeichnet,** daß das resorbierbare Material Polylactid ist.

4. Marknagel (1) nach einem der vorhergehenden Ansprüche, **dadurch gekennzeichnet,** daß sein Nagelkopf (2) aus metallischem Material besteht und daß eine Hülse (9) den resorbierbaren Bereich der Nagelspitze (4) mit dem Nagelkopf (2) formschlüssig verbindet.

5. Marknagel (1) nach Anspruch 4, **dadurch gekennzeichnet,** daß die Hülse (9) aus Titan oder einer Titanlegierung besteht.

6. Marknagel (1) nach einem der vorhergehenden Ansprüche, **dadurch gekennzeichnet,** daß die Trennstelle zwischen Nagelkopf (2) und resorbierbarem Bereich der Nagelspitze (4) unmittelbar über dem Bereich der Verriegelungsverschraubung vorgesehen ist.

7. Marknagel (1) nach einem der vorhergehenden Ansprüche, **gekennzeichnet** durch eine durchgehende zentrische Bohrung.

## Claims

1. Intramedullar nail (1) for operative treatment of fractures of a long tubular bone, which nail is fixable by means of fixation screws (7, 8),
**characterized in that**
said intramedullar nail (1) consists of an absorbable material in its nail tip section (4) only.

2. Intramedullar nail (1) according to claim 1, **characterized** in that at least one of said fixation screws (7, 8) consists of an absorbable material.

3. Intramedullar nail (1) according to one of the preceding claims, **characterized in that** said absorbable material is polylactate.

4. Intramedullar nail (1) according to one of the preceding claims, **characterized in that** the nail head (2) consists of a metallic material, and that a sleeve (9) connects the absorbable nail tip (4) section firmly with said nail head (2).

5. Intramedullar nail (1) according to claim 4, **characterized in that** said sleeve (9) consists of titanium or a titanium alloy.

6. Intramedullar nail (1) according to one of the preceding claims, **characterized in that** the interface between nail head (2) and the absorbable nail tip section (4) is directly above an area in which said fixation screws are inserted.

7. Intramedullar nail (1) according to one of the preceding claims, **characterized by** a throughgoing, concentric bore.

## Revendications

1. Clou intramédullaire (1) qui peut être immobilisé par des vis de verrouillage (7,8), pour le traitement chirurgical de fractures d'os tubulaires longs, caractérisé en ce que le clou intramédullaire est composé d'une matière résorbable exclusivement dans la région de sa pointe (4).

2. Clou intramédullaire (1) selon la revendication 1, caractérisé en ce qu'au moins une des vis de verrouillage (7,8) est faite d'une matière résorbable.

3. Clou intramédullaire (1) selon l'une des revendications précédentes, caractérisé en ce que la matière résorbable est un polyactide.

4. Clou intramédullaire (1) selon l'une des revendications précédentes, caractérisé en ce que sa tête (2) est faite d'une matière métallique et en ce qu'une douille (9) réunit la région résorbable de la pointe (4) du clou à la tête (2) du clou avec une liaison par sûreté de forme.

5. Clou intramédullaire (1) selon la revendication 4, caractérisé en ce que la douille (9) est composée de titane ou d'un alliage de titane.

6. Clou intramédullaire (1) selon l'une des revendications précédentes, caractérisé en ce que la jonction entre la tête (2) du clou et la région résorbable de la pointe (4) du clou est prévue immédiatement au-dessus de la région du vissage de verrouillage.

7. Clou intramédullaire (1) selon l'une des revendications précédentes, caractérisé par un alésage central traversant.
